# EUROPEAN PATENT SPECIFICATION

(11) **EP 1 039 931 B1**
(45) Date of publication and mention of the grant of the patent: **27.04.2005**
(21) Application number: 98960577.9
(22) Date of filing: 30.11.1998
(51) Int. Cl.: A61K 39/395

(54) **MULTIVALENT RECOMBINANT ANTIBODIES FOR TREATING HRV INFECTIONS**
MULTIVALENTE REKOMBINANTE ANTIKÖRPER ZUR BEHANDLUNG VON HRV INFEKTIONEN
ANTICORPS DE RECOMBINAISON MULTIVALENTS DESTINES AU TRAITEMENT D'INFECTIONS DUES AU RHINOVIRUS HUMAIN (HRV)

(30) Priority: 01.12.1997 US 67119 P; 24.04.1998 US 83046 P; 25.06.1998 US 90632 P
(43) Date of publication of application: 04.10.2000
(73) Proprietor: Fang, Fang, San Diego, CA 92130 (US)
(72) Inventor: Fang, Fang, San Diego, CA 92130 (US)
(74) Representative: Baldock, Sharon Claire
(86) International application number: PCT/US1998/025422
(87) International publication number: WO 1999/027964

(56) References cited:
- EP-A2- 0 459 577
- KARUSH, F. ET AL: "Multivalence and affinity of antibody" INT. ARCH. ALLERGY APPL. IMMUNOL. (1973), 45(1-2), 130-2 , XP000960564
- PACK, PETER ET AL: "Tetravalent miniantibodies with high avidity assembling in Escherichia coli" J. MOL. BIOL. (1995), 246(1), 28-34 , XP000960570
- TERSKIKH A.V. et al., "Peptabody: A New Type of High Avidity Binding Protein", PROC. NATL. ACAD. SCI. USA, March 1997, Vol. 94, pages 1663-1668, XP002917159
- WELPLY J.K. et al., "A Peptide Isolated by Phage Display Binds to ICAM-1 and Inhibits Binding to LFA-1", PROTEINS: STRUCTURE, FUNCTION AND GENETICS, 1996, Vol. 26, pages 262-270, XP002917160
- HOFER F. et al., "Members of the Low Density Lipoprotein Receptor Family Mediate Cell Entry of a Minor-Group Common Cold Virus", PROC. NATL. ACAD. SCI. USA, March 1994, Vol. 91, pages 1839-1842, XP002917161
- HODITS R.A. et al., "An Antibody Fragment from a Phage Display Library Competes for Ligand Binding to the Low Density Lipoprotein Receptor Family and Inhibits Rhinovirus Infection", THE JOURNAL OF BIOLOGICAL CHEMISTRY, 13 October 1995, Vol. 270, No. 41, pages 24078-24085, XP002917162
- KAISER ET AL: 'In vitro activity of pleconaril and AG7088 against selected serotypes and clinical isolates of human rhinoviruses' ANTIVIRAL RESEARCH vol. 47, 2000, pages 215 - 220
- HAYDEN ET AL: 'Phase II, randomised, double-blind, placebo-controlled studies of Ruprintvir nasal spray 2-percent suspension for prevention and treatment of experimentally induced rhinovirus colds in healthy volunteers' ANTIMICROBIAL AGENTS AND CHEMOTHERAPY vol. 47, no. 12, December 2003, pages 3907 - 3916

## Description

### FIELD OF THE INVENTION

This invention relates to high affinity multivalent recombinant antibodies, multivalent peptides, and their use in preventing and treating viral infections and related diseases.

### BACKGROUND OF THE INVENTION

Human rhinoviruses (HRV) are among the most frequently occurring human pathogens. HRV are responsible for majority cases of the common cold (Stanway, G. 1994, "Rhino viruses". In: Webster R. G. (eds) Encyclopedia of Virology, New York: Academic Press, pp. 1253-1259; Sperber, S. J. and Hayden, F. G. 1988, Antimicrob. Agents Chemother. 32:409-419). In addition, HRV are a leading cause of upper respiratory tract infection and otitis media, *i*.*e*., ear infection (Arola, M. *et al*., 1990, Pedia 86:848-855).

Recurrent acute otitis media (AOM) is a prevalent infectious disease among young children and results in more than 8 million pediatric office visits each year. Teele *et al*., 1989, J. Infect Dis. 160:83-94 found that 62% of children at age one have had at least one episode of AOM and 17% have had three or more. By age three, more than 80% of children have experienced otitis media and more than 40% have had three or more episodes. The annual treatment costs for AOM has been estimated at more than $3.5 billion in the United States (Stool, S.E, and Field, M.J., 1989, Pediatr. Infect Dis. J. 8:S1 1-14).

At least 115 distinct serotypes of HRV have been reported (Uncapher *et al*., 1991, Virology 180:814-817). Because of the large number of HRV serotypes, little immunological protection is afforded to a human subject by prior exposure to one or a few heterologous serotypes. The genetic heterogeneity of HRV accounts for the high incidence of rhinovirus infection.

Commercially available drugs only treat the symptoms of the common cold and AOM. They can not prevent rhinovirus infections. Prophylactics for the common cold are desirable, especially for young children who are most susceptible to the common cold and ear infections.

### SUMMARY OF THE INVENTION

The present invention relates to the generation of high affinity multivalent recombinant antibodies and multivalent peptides that bind to cellular receptors for rhinovirus and especially HRV. Specifically, it is directed to multivalent recombinant antibodies and multivalent peptides against the HRV binding sites on human ICAM-1 molecules. This invention also relates to methods of using these antibodies and peptides to block the receptors so as to prevent and treat HRV infections and associated diseases and pathological conditions, including, but not limited to, the common cold, otitis media, asthma, bronchitis and sinusitis.

A "human rhinovirus" is accorded the definition provided in Hamparian *et al*., 1987, Virology 159:191-192, and specifically includes all human serotypes of rhinovirus catalogued in Hamparian *et al*., 1987, Virology 159:191-192, and additional serotypes of HRV which have been identified and will be identified thereafter.

A "recombinant antibody" in this invention contains the heavy chain variable domain (V_{H}) and light chain variable domain (V_{L}) of an antibody linked together in a single chain. The recombinant antibody specifically recognizes a cellular receptor for HRV, including, but not limited to, the major receptor ICAM- 1, and reduces or prevents binding of HRV to human host cells.

A "multivalent recombinant antibody" in this invention includes any multimeric configuration of two or more, and especially three or more recombinant antibodies. Specifically; it includes, trivalent, tetravalent and pentavalent recombinant antibodies. Multiple antigen binding domains can be coupled to an inert polymer support, including, but not limited to, nitrocellulose, PVDF, DEAE, amino dextran, and lipid polymers. The multivalent antibody contains multiple units polymerized through their polymerization domains. Each unit contains a Fv fragment and a polymerization domain linked together (preferably expressed as a single chain polypeptide). The polymerization domains are capable of binding to each other by virtue of containing one or more peptide fragments having affinity for other peptide fragments of the same or substantially the same amino acid sequence. The polymerization domain determines the configuration of the multimeric protein complex (*e*.*g*., as dimer, trimer, tetramer, pentamer, or others). Examples of polymerization domains include, but are not limited to, coiled-coil domains such as described and defined in Lupas *et al*., 1991, Science 252:1162-1164, and alpha-helical sequences such as described in Eisenberg, D. *et al*., (1986) Protein 1:16-22, Ho and DeGrado (1987) J.A.Chem.Soc. 109:6751-6758, Regan and DeGrado, (1988) Science 241:976-978, and Hill *et al*., (1990) Science 249:543-546. A polymerization domain can be a naturally occurring peptide or can be designed and synthesized artificially. A naturally occurring polymerization domain may be modified to generate other polymerization domains (*see, e*.*g*., methods described in Harbury *et al*. Science 262:1401-1407, 1993). The coiled-coil domain may come from the leucine zipper region of transcription factor GCN4, the tetramerization domain of p53, or the N-terminal residues (aa 20-80) of cartilage oligomeric matrix protein (*i*.*e*., COMP). The alpha-helical sequences may contain single helices or helix-turn-helix as summarized in Plückthun and Pack, (1997) Immunotechnology 3:83-105. The antigen binding sites or scFv fragments recognize cellular receptors for rhinoviruses, including, but not limited to, ICAM-1, LDL receptor and its related protein αMR/LRP. They recognize the rhinovirus binding sites on ICAM-1. The antigen binding sites or scFv fragments of a multivalent recombinant antibodies may recognize the same target (*e*.*g*., either ICAM-1 or LDLR) or two or more different targets (*e*.*g*., some recognize ICAM-1. while others recognize LDLR).

A "multivalent peptide" includes any multimeric configuration of two or more, and especially three or more peptides. Specifically, it includes bivalent, trivalent, tetravalent and pentavalent peptides. Multiple antigen binding domains can be coupled to an inert polymer support, including, but not limited to, nitrocellulose, PVDF, DEAE, amino dextran, and lipid polymers. The multivalent peptides are in the form of tandem repeats in which peptides are joined directly or via linker sequences (*e*.*g*., by recombinant DNA technology). The multivalent peptide may, contain multiple units polymerized through their polymerization domains. Each unit contains a single peptide (or peptides in tandem repeats) and a polymerization domain linked together (preferably expressed as a single chain polypeptide). The polymerization domains are capable of binding to each other by virtue of containing one or more peptide fragments having affinity for other peptide fragments of the same or substantially the same amino acid sequence. The polymerization domain determines the configuration of the multimeric protein complex (e.g., as dimer, trimer, tetramer, pentamer, or others). Examples of polymerization domains include, but are not limited to, coiled-coil domains such as described and defined in Lupas *et al*., 1991, Science 252:1162-1164, and alpha-helical sequences as described and defined in Eisenberg, D. *et al*., (1986) Protein 1: 16-22, Ho and DeGrado (1987) J.A.Chem.Soc. 109:6751-6758, Regan and DeGrado, (1988) Science 241:976-978, and Hill *et al*., (1990) Science 249:543-546. A polymerization domain can be a naturally occurring peptide or can be designed and synthesized artificially. A naturally occurring polymerization domain may be modified to generate other polymerization domains (*see* Harbury *et al*., Science 262:1401-1407, 1993). The coiled-coil domain may come from the leucine zipper region of transcription factor GCN4, the tetramerization domain of p53, or the N-terminal residues (aa 20-80) of cartilage oligomeric matrix protein. The alpha-helical sequences may contain single helices or helix-turn-helix as summarized in Pluckthun and Pack, (1997) Immunotechnology 3:83-105.

HRV bind to ICAM-1 in four regions. In preferred embodiments, the multivalent peptides bind to three or more (preferably four or more) amino acids in the following regions of human ICAM-1. These are referred to as the target sequences or target peptides hereinafter:
Target No. 1: Residues 1-5: QTSVS
Target No. 2: Residues 24-29: SCDQPK
Target No. 3: Residues 40-49: KELLLPGNNR
Target No. 4: Residues 70-77: PDGQSTAK

The multivalent recombinant antibodies and peptides of this invention bind to cellular receptors for rhinovirus with high affinity. In a preferred embodiment, they have an apparent affinity constant for the cellular receptors of no less than 10⁸ M⁻¹. They may have an apparent affinity constant for the cellular receptors of no less than 10⁹M⁻¹. They may have an apparent affinity constant for the cellular receptors of no less than 10¹⁰ M⁻¹.

By "apparent affinity constant" is meant the ratio of [Ab-Ag]/[Ab][Ag] when the antibody-antigen binding reaction reaches equilibrium. [Ab-Ag], [Ab], [Ag] are the concentrations of antibody-antigen complex, free antibody, and free antigen, respectively. The apparent affinity constant of multivalent peptides is measured in the same way as the antibodies.

This invention also features formulations containing a pharmaceutically effective amount of an aforesaid multivalent recombinant antibody and a pharmaceutically acceptable carrier.

By "pharmaceutically effective" is meant the ability to prevent, treat, reduce or cure rhinovirus infection or one or more clinical symptoms of rhinovirus infection.

In a preferred embodiment, the composition comprises an antibody having binding affinity for ICAM-1 and preferably a multivalent recombinant antibody (mvAb) having binding affinity for ICAM-1.

A composition comprise a first mvAb having binding affinity for ICAM-1 and a second mvAb having binding affinity for LDLR.

A composition may comprise peptides (preferably multivalent peptides) having binding affinity for two or more (or three or more) HRV binding regions in ICAM-1.

A composition may comprise multivalent peptides having binding affinity for no more than two HRV binding regions in ICAM-1.

The above identified multivalent recombinant antibodies and multivalent peptides may be used to prevent and treat rhinovirus infections and associated diseases or pathological conditions in mammals, especially in humans, including, but not limited to, the common cold, otitis media, bronchitis and sinusitis. In addition, the multivalent recombinant antibodies and peptides against ICAM-1 may also be used to prevent the infection of other viruses that are known to bind to ICAM-1, such as Coxsackie A virus. Furthermore, the multivalent recombinant antibodies against ICAM-1 may be delivered intravenously to block the interaction between LFA-1 and ICAM-1 as a way to control the immunological response relating to inflammation.

Other features and advantages of the invention will be apparent from the following detailed description of the invention, and from the claims.

### DETAILED DESCRIPTION OF THE INVENTION

HRV infect human upper respiratory tract by attaching to and then entering the epithelial cells lining the nasal cavity and nasopharynx. The attachment is mediated by specific interaction between HRV virions and receptors on the surface of epithelial cells.

Based on their selection of cellular receptors, HRV are grouped into two families (except for HRV-87): the major group and the minor group (Uncapher *et al*., 1991, Virology 180:814-817). The major group contains at least 91 serotypes, all of which recognize a receptor identified as the intercellular adhesion molecule 1 (ICAM- 1) (Greve *et al*., 1989, Cell 56:839-847; Staunton *et al*., 1989, Cell 56:849-853; Tomassini, 1989, Proc. Natl. Acad. Sci. USA 86:4907-4911). The minor group contains about 10 serotypes, and their cellular receptor is the low-density lipoprotein receptor (LDLR) and a related protein "alpha macroglobulin receptor/LDLR-related protein (αMR/LRP) (Hofer *et al*., 1994, Proc. Natl. Acad. Sci. USA 91:1839-1842).

ICAM-1 is a member of the immunoglobulin supergene family and contains five homologous immunoglobulin-like domains defined by amino acids 1-88, 89-185, 186-284, 285-385, and 386-453 (Staunton *et al*., 1988, Cell 52:925-933). ICAM-1 is also the cell surface ligand for the lymphocyte function-associated antigen-I (LFA-I). A soluble form of ICAM-1 has been detected in normal human serum (Rothlein *el al*., 1991, J. Immunol. 147:3788-3793). The expression of ICAM-1 is upregulated by inflammatory cytokines in activated leukocytes, endothelium and epithelium. Two other homologous molecules, ICAM-2 and ICAM-3, have similar cellular distributions like ICAM-1 and also bind to LFA-1.

ICAM-1 has distinct binding sites for rhinovirus and LFA-1 (Staunton *et al*., 1990, Cell 61:243-254). The rhinovirus-binding site has been mapped to the N-terminal Ig-like domain (amino acids 1-88) (Staunton *et al*., 1990, Cell 61:243-254; McClelland *et al*., 1990, Proc. Natl. Acad. Sci. USA 88:7993-7997).

The extracelluar portion of the ICAM-1 molecule has five immunoglobulin-like domains (D1 -D5). D1 contains the primary binding site for rhinoviruses as well as the binding site for its natural ligand, *i*.*e*., lymphocyte function-associated antigen 1 (LFA- 1). Distinct amino acid residues are involved in the binding of ICAM-1 to HRV and LFA-1. The HRV contacting sites in D1 include residues 1, 2, 24-29, 40-49, and 70-77 (Staunton *et al*., (1988) Cell 52:925-933; Staunton *et al*., (1990) Cell 11:243-254; McClelland *et al.,* (1991) Proc. Natl. Acad. Sci. USA 88:7993-7997; Lineberger *et al*., (1990) J. ViroI 64:2582-2587; and Olson *et al*., (1993) Proc. Natl. Acad. Sci. USA 90:507-511).

The three-dimensional structure of D1 -D2 portion of the ICAM-1 molecule has been revealed by Casasnovas *et al*., (1998) Proc. Natl. Acad. Sci. USA 95:4134-4139; and Bella *et al*., (1998) Proc. Natl. Acad. Sci. USA 95:4140-4145. The tip of ICAM-1 molecule has three loops: BC (aa 23-29), DE (aa 43-48) and FG (aa 69-72). The three loops penetrate deep into the canyon on the surface of HRV. Antibodies or peptides that bind to these regions of the ICAM-1 molecule may mask the HRV binding site and prevent HRV from binding to ICAM-1.

Whereas a single peptide may not have the affinity required to prevent the interaction between HRV and ICAM-1, multivalent peptides which bind to the HRV binding regions of human ICAM-1 molecule can be used to prevent HRV infection in human and prevent and treat the common cold. It has been shown that multivalency can increase the affinity of a peptide by 105 times (Terskiskh *et al*., 1997, Proc. Natl. Acad. Sci. USA 94:1663-1668).

The physiological function of LDLR is to bind plasma LDL, a cholesterol transport protein, initiate the internalization of the LDL, and supply cholesterol to cells (Daniel *et al*., 1983, J. Biol. Chem. 258:4606). The LDL receptors on the surface of nasal epithelial cells are probably of little importance to the normal physiological functions of these mucosal cells since the nasal mucosa usually do not have access to plasma LDL.

One strategy of preventing HRV infection is to administer soluble ICAM-1 and LDLR as decoys to mask the receptor binding sites of HRV (U.S. Patent Serial Nos. 5,589,453, 5,674,982 and 5,603,932). The soluble forms of ICAM-1 (sICAM-1) have been shown to inhibit virus-receptor binding and decrease the virus infectivity (Marlin *et al*., 1990, Nature 344:70-72; Greve *et al*., 1991, J. Virol. 65:6015-23). Nasal spray made with soluble ICAM-1 has been shown to protect chimpanzees against HRV challenge (Huguenel *et al*., 1997, American J. Resp. Critical Care Med. 155(4):1206-10). However, the protection afforded by sICAM-1 is very short (approximately 30 minutes) because the interaction between sICAM-1 and HRV is weak and the mucociliary clearance mechanism removes sICAM-1 molecules rather quickly (Arruda *et al*., 1992, Antimicrobial Agents and Chemotherapy 36:1186-91; Illum, 1991, Trend in Biotech 9:284-289). Such shortcomings limit the use of sICAM-1 as a prophylactic against HRV infections.

Another strategy for preventing HRV infection is to block the HRV binding site on ICAM-1 with a monoclonal antibody (mAb) (Lineberger *et al*., 1990, J. Virol. 64:2582-2587). It has been shown that a monoclonal antibody against ICAM-1, 1A6, remained bound to ICAM-1 molecule for two days in a cell culture, and the association withstood multiple washes (Colonno *et al*., 1989, in Molecular Aspects of Picornavirus Infection and Detection (Semler BL and Ehrenfeld E, eds) pp. 169-178, American Society for Microbiology, Washington, D.C.). No adverse effect on cellular functions was observed when a 1,000-fold excess of antibody against ICAM-1 was cultured with HeLa cells (Colonno *et al*., 1986, J. Virol. 57:7-12). In addition, intranasal administration of the antibody ICAM-1 in chimpanzees for up to one year did not produce detectable local or systemic toxicity (Hayden *et al*., 1988, Antiviral Res. 9:233-247). The same group also showed that intranasal administration of 1A6 (1 mg/subject in 10 applications, -3 to +36 hours) delayed the onset of rhinovirus colds in human volunteers by up to 2 days.

However, 1A6 did not reduce overall infection or illness rates (Hayden *et al*., 1988, Antiviral Res. 9:233-247. Casasnovas *et al*., 1995, J. Bio. Chem. 270:13216-24 showed that the dissociation rates were similar between mAbs/ICAM-1 and HRV/ICAM-1 (1.67x10⁻³s⁻¹ translating to a t_{1/2} of receptor-ligand bond of 6.9 minutes).

A single chain antibody has been cloned from 1A6 and has been shown to be able to block the HRV infection of cultured cells albeit at an efficacy lower than that of the original mAb (Condra *et al*., 1990, J. Bio. Chem. 265 :2292-95).

This invention encompasses Applicant's appreciation that the prophylactic effect of 1A6 and its corresponding single chain mAb is limited by their functional affinity (avidity) to ICAM-1. It is imperative to decrease the dissociation rate of the receptor-antibody complex (as measured by Kd) in order to increase the avidity of antibodies to cellular HRV receptors.

### Preventing HRV infection with recombinant multivalent antibodies and/or multivalent peptides having high avidity for ICAM-1 and/or LDLR

Certain native proteins increase their affinity to ligands through multimeric complexes. For example, although individual IgG molecules bind the complement factor Clq at a low affinity (100 mM), the same IgG molecules in clusters bind Clq at much higher affinities (about 1 mM and 3 nM, respectively, for IgG dimers and tetramers, Male *et al*., 1987, in Advanced Immunology, eds. Male; D., Champion, B. and Cooke, A. (Gower Medical, London), pp. 2.1-2.13.).

Within the scope of this invention, Applicant prepares multivalent recombinant antibodies against cellular receptors of rhinoviruses in order to utilize multivalency to achieve high affinity to the receptors and to stabilize the association between the antibodies and the receptors by decreasing the dissociation rate. A recombinant antibody with four (tetravalent) or five (pentavalent) binding sites will have much higher avidity to its target than a monoclonal antibody (bivalent) or a single chain antibody (monovalent). Such multivalent recombinant antibodies against ICAM-1 and LDLR are much more effective in preventing HRV infection than monoclonal antibodies and single chain antibodies. Likewise, a pentavalent peptide has 0 106 times higher avidity than a single peptide and will be able to block HRV binding sites on ICAM-1 much more effectively.

### Methods of making multivalent recombinant antibodies and/or multivalent peptides

A multivalent recombinant antibody (mvAb) can be made by linking the antigen binding domain of an antibody (*e.g.*, a single chain Fv fragment (scFv)) with a protein domain which polymerizes automatically, including, but not be limited to, the coiled-coil sequences and alpha-helical sequences in proteins. The antigen binding domain and the polymerizing domain can be connected through a synthetic linker. Recombinant antibodies can also be multimerized by adsorption or chemical coupling to a support made of a variety of inert polymers, including, but not limited to, nitrocellulose, PVDF, DEAE, amino dextran, and lipid polymers. A multivalent recombinant antibody (mvAb) made by coiled-coil protein domains or alpha-helical domains can be further polymerized by coupling to inert polymers to prepare a molecular complex of higher avidity. Multivalent peptides can be made in a similar way as the multivalent antibodies by replacing the scFv fragment with either a single peptide or a tandem repeat of peptides.

Coiled-coil domains have been identified in more than 200 proteins in GenBank (Lupas *et al*., 1991, Science 252:1162-1164), including the leucine zipper transcription factors and the cartilage oligomeric matrix protein (COMP). Coiled-coil proteins have a characteristic seven-residue repeat, and the seven positions are designated as a through g, (*a.b.c.d.e.f.g*)ₙ, with hydrophobic residues at positions *a* and *d* and polar residues generally elsewhere.

The methods described and disclosed in the following references can be used for the purposes of this invention in making tetrameric and pentameric recombinant antibodies.

Harbury *et al*., 1993, Science 262:1607-1407 prepared stable trimeric and tetrameric complexes through simultaneous changes in positions a and d of the seven-residue repeat in the leucine zipper domain of a transcription factor GCN4 (a coiled-coil sequence). In addition, a parallel tetrameric-helix complex was formed when the hydrophobic residues in position a was changed to leucine and the leucine residues in position d was changed to isoleucine.

Pack *et al*., 1995, J. Mol. Biol. 246:28-34 made a tetravalent miniantibody against phosphoryl choline in the periplasm of *E*. *coli* by linking a single-chain Fv fragment of phosphoryl choline-binding antibody to the modified leucine zipper domain ofGCN4. This tetravalent miniantibody exhibited higher avidity than that of the bivalent construct.

Rheinnecker *et al*., 1996, J. Immunol. 157:2989-2997 made a tetravalent miniantibody against a tumor-associated carbohydrate antigen Lewis Y by linking the scFv fragment with the tetramerization domain of human transcription factor p53 (amino acid 319 to 360). The multivalency lowered the dissociation rate of antigen-antibody complex by more than two orders of magnitude.

Cartilage oligomeric matrix protein (COMP) is a pentameric glycoprotein of the thrombospondin family found in cartilage and tendon. Self-association of COMP is achieved through the formation of a five-stranded α-helical bundle that involves 64 N-terminal residues (from amino acid 20 to 83). The complex is further stabilized by the interchain disulfide bonds between cysteines 68 and 71 (Efimov *et al*., 1996, Proteins 24:259-262).

To increase the affinity of a synthetic peptide to its ligand, Terskish *et al*., 1997, Proc. Natl. Acad. Sci. USA 94:1663-1668 prepared a pentameric multivalent binding molecule by linking the peptide with the coiled-coil assembly domain of the COMP (residues 26-80). This homopentamer exhibits an avidity to its ligand at a level that is 2x10⁵ folds higher than that of the original peptide.

Therefore, a pentavalent recombinant antibody or a pentavalent peptide can. be generated by linking the scFv fragment or a peptide with the coiled-coil domain of COMP.

### Example 1: Preparation of Multivalent Recombinant Antibodies Against ICAM-1 and LDL Receptor

### A. Amplification and purification of rhinoviruses

Rhinoviruses are grown, purified and assayed as previously described (Abraham, G. *et al*., 1984, J. Virol. 51:340; Greve *et al*., 1989, Cell 56:839-847; U.S. Patent 5,603,932). They are plaques purified and isolated from lysates of infected HeLa cells by polyethylene glycol precipitation and sucrose gradient centrifugation. Purity of the viral preparation is assessed by SDS-PAGE analysis and electron microscopy. Infectivity is quantitated by a limiting dilution assay as described by Minor, P.D., Growth, assay and purification of picomaviruses, In Virology: A Practical Approach, B. W. J. Mahy, ed (Oxford: IRL Press), pp. 25-41.

Specifically, HRV14, HRV3, HRV2 and HRV49 are chosen for this study, and all can be obtained from American Tissue Culture Collection. HRV14 and HRV3 bind to the major receptor ICAM-1 while HRV2 and HRV49 bind to the minor receptor LDLR.

### B. Generation of monoclonal antibody against ICAM-1 and LDLR

HeLa cells are used to immunize BALB/c mice because both ICAM-1 and LDL receptor are expressed on the surface thereof. The procedure is described in U.S. Patent 5,674,982. Alternatively, polypeptides containing the rhinovirus-binding domain of ICAM-1 or LDLR can be used to immunize mice.

Briefly, 10⁷ HeLa cells in 0.5 ml of phosphate-buffered saline (PBS) are injected into the peritoneal cavity of the BALB/c mice three times at three-week intervals. Two weeks later the mice are bled and the sera are tested for their capacity to protected HeLa cells from being infected by HRV14 and HRV2. Positive mice are boosted by a final injection of 10⁷ HeLa cells. Three days later, the spleen cells are fused with myeloma cells to produce hybridoma. This can be carried out through well-known techniques, such as those described in U.S. Patent 4,196,265 or in Harlow and Lane, 1988, Antibodies, A Laboratory Manual, Cold Spring Harbor Laboratory.

The hybridoma is then screened as described in U.S. Patent 5,674,982. Briefly, 100 ml of supernatant from each hybridoma clone is incubated with 3x10⁴ HeLa cells in 96-well plated for 1 hr at 37°C; the cells are then washed with PBS, and a sufficient amount of HRV14 or HRV2 are added to give complete cytopathic effect in 24-36 hr. Wells that are protected from infection (positive) are scored at 36 hr. The corresponding positive hybridoma are undergo several round of cloning by limiting dilutions in 96-wells until all of the hybridoma containing wells are positive.

ICAM-1 and LDLR used to raise antibody in this invention can be of any species origin as long as the antibody raised is able to reduce the binding of HRV to their human host cells.

### C. Cloning the single chain Fv (scfv) region of the mAb and humanizing the antibody

Single chain Fv fragments against ICAM-1 and LDLR are cloned from hybridomas against each antigen.

One way (a preferred way) of cloning the VH and VL fragments is to amplify them by polymerase chain reaction (PCR) from a hybridoma directed against ICAM-1 or LDLR. Alternatively, the Fab fragment of the desired antibody can be cloned from a combinatorial iminunoglobulin library in phage λ by panning using the purified ICAM-1 and LDLR as the antigens (Kang *et al*., 1991, Methods 2:111-118; Barbas and Lerner, 1991, Methods 2:119-124). The polypeptides of VI-I and VL can be connected via a synthetic linker to form a single-chain Fv fragment (scFv), or one scFv fragment can dimerize with another to form either diabodies (Holliger *et al*., 1993, Proc. Natl. Acad. Sci. USA 90:6444-6448) or chelating recombinant antibodies (CRAbs) (Neri *et al*., 1995, J. Mol. Biol. 246:367-373).

The amino acid sequences of the VH and VL domains can be modified based on the original antibody to increase the affinity to antigens or improve the yield of production in E coli or yeast. The origin of the antibody can be of any mammals, including, but are not limited to, human, mouse, rat and rabbit. When the original antibody is isolated from a species other than human, the VH and VL domains of the antibody can be humanized, such as using the methods in U.S. Patent 5,530,101.

Total RNA is isolated from each hybridoma and is converted into cDNA by AMV reverse transcriptase. The VH and VL genes are amplified by PCR using different combinations of degenerate primers in the Ig-Primer kit (Novagen, WI) according to the manufacturer's protocol, or using the primers as described in Larrick & Fry, 1991, Methods 2:106-110. The resulting VH and VL fragments are cloned into a TA cloning vector (Invitrogen, CA) and sequenced. The sequences of multiple clones are compared and the consensus sequences are used to construct the scFv fragments. The VH and VL genes are linked by an artificial linker (GGGGS)₃ to form scFv fragment [VH-GGGGS]₃-VL] as in Batra *et al*., 1990, and the scFv fragment is subcloned into a pBlueScript vector (Stratagene, CA). Methods of making svFv fragment are also described in U.S. Patents 5,571,894 and 5,608,039.

The cloned scFv sequence can be humanized to make it less immunogenic or nonimmunogenic to a human host. The humanization of antibody can be carried out as described in U.S. Patent 5,530,101. The sequences of scFv fragments can also be modified. to increase the yield of production while still maintaining their antigenic specificity, such as changing the genetic codons or including a signal sequence as described in U.S. Patent 5,648,237.

### D. Constructing multivalent recombinant antibody genes

Methods of making multivalent recombinant antibodies are summarized in Plückthun and Pack, (1997) Immunotechnology 3:83-105, incorporated by reference herein.

A pentavalent recombinant antibody is preferred. scFv fragments are linked with the pentamerization domain of COMP via a hinge region to give rise to the following fragment: scFv-hinge[(PQ)₂PK(PQ)₄PKPQPK(PE)₂]-Pentamerization domain (COMP aa 28-72).

The pentamerization domain of COMP is amplified from plasmid p3b-COMP (Efimov *et al*., 1994, FEBS letters 341:54-58) by PCR (Tomschy *et al*., 1996, EMBO J. 14:3507-3514). The amino acid sequence of this COMP domain can be modified to increase the stability of the complex. For example, the Lys-29 and Ala-30 can be changed to cysteine residues (Terskish *et al*., 1997, Proc. Natl. Acad. Sci. USA 94:1663-1668).

There are numerous sequences known to those skilled in the art as suitable for the hinge region. Therefore, the one listed here is only an example. The COMP pentamerization domain can be linked with the hinge and scFv by PCR and ligation using the standard molecular biology techniques, The whole fragment is subcloned into pTrc/His vector (Invitrogen, CA) to generate a bacteria expression plasmid pTrc/scFv-COMP.

### E. Expressing multivalent recombinant antibodies in E. coli

The scFv-comp protein is expressed in E. coil as described in Terskiskh *et al*., 1997, Proc. Natl. Acad. Sci. USA 94:1663-1668. Briefly, *E*. *coli* transformed with the plasmid pTrc/scFv-COMP is grown in culture until OD600 = 0.5, then 1 mM isopropyl b-D-thiogalactoside is added to induce protein synthesis, followed by a 4 hr incubation at 30°C. Bacteria are harvested by centrifugation. lysed by three-rounds of freezing/thawing. The recombinant antibody protein is purified from the bacterial lysate by a nickel column (Qiagen).

The biological function of the multivalent recombinant antibodies (mvAb) is then examined by the receptor-binding assay and the cell protection assay described blow.

### Materials and Methods

1. The HRV major receptor ICAM-1 protein can be prepared from Hela cells as described in U.S. Patent 5,589,453. The minor receptor LDLR can be purified from Hela cells as described in U.S. Patent 5,603,932 or Schneider *et al.,* 1985, Methods Enzymology 109:405-417. Alternatively, soluble LDLR fragment can be expressed and purified from Sf9 insect cells as described in Marlovits *et al*., (1998) FASEB 12:695-703.
2. Preparation of ³⁵S-methionine labeled human rhinovirus serotypes 14 and 2 (HRV14 and HRV2)
   The procedure is carried out as described in U.S. Patent 5,603,932. Briefly, four HeLa cell monolayers in 165cm² Petri dishes are infected with an MOI (multiplicity of infection) of 40 with HRV14 and HRV2 for 1 hr at 34°C in methionine-free MEM medium (Gibco) with 2% dialyzed fetal calf serum. The cells are then washed twice with PBS and incubated at 34°C in fresh medium.
   After 3 hr, 1 mCi ³⁵S-methionine (Amersham) is added to each monolayer and incubation is continued for 24 hr. The medium of the infected cells is then cleared by centrifugation for 30 mm at 4,5000xg. The supernatant from the centrifugation is centrifuged for 2 hr at 140,000xg. The virus pellet is resuspended in Tris/EDTA and further purified over a 10-30% sucrose gradient. The individual fractions of the gradient are analyzed by 12% SDS-PAGE gel. The pure virus fractions are combined and stored in the presence of 1% bovine serum albumin (BSA) at 4°C.
3. Receptor Binding Assay
   To demonstrate that the multivalent recombinant antibodies (mvAb) can block the binding of HRV to ICAM-1 and LDLR, various amount of mvAb is used in this assay to inhibit the binding of ³⁵S HRV14 and ³⁵S HRV2 to previously immobilized ICAM-1 and LDLR. This assay is based on the Solution Binding Assay described in U.S. Patent 5,674,982 with minor modifications. Purified ICAM-1 or LDLR is diluted in Tri/NaCl buffer and allowed to adsorb to the walls of a microtiter plate. Each well of the microtiter plate is then further blocked with 10 mg/mi BSA and then washed with PBS. Specific mvAbs are diluted with 0.1% Triton X-1 00/1 mg/ml BSA/Tris/Nacl, added to each well precoated with either ICAM-1 or LDLR and allowed to incubate for 1 hr at 37 C. The same buffer without mvAb is the positive control. The unbound mvAb is then washed off with PBS. Approximately 20,000 cpm of ³⁵S HRV14 (to ICAM-1 precoated plates) and ³⁵S HRV2 (to LDLR precoated plated) are added. to the each mvAb treated well and allowed to incubate for 1 hr at 37°C. The plates are washed and the bound radioactivity determined. Less radioactivity in the mvAb treated wells is expected. Total inhibition of ³⁵S HRV binding can be achieved if enough mvAb is used to completely block the precoated ICAM-1 and LDLR.
4. Cell protection assay
   The assay described in Colonno *et al*., 1986, J. Virol. 57:7-12 is used to demonstrate that mvAb against ICAM-1 and LDLR can protect host cells from HRV infection.
   HeLa cells are plated in 48-well plates (Costar) at 1.5x10⁵ cells per well and incubate over night to generate confluent monolayers. Duplicate monolayers are treated with various amount of each mvAb and incubated for 1 hr at 37°C. HRV14, HRV3, HRV2 and HRV49 are then applied to each well at MOI of 0.1 to 1 and allowed to incubate overnight at 37°C. The next day, monolayer is checked for cytopathic effect with a light microscope. Wells without the treatment of mvAbs but are infected by the HRV are used as controls. Less cytopathic effect is expected in the mvAb treated cells.
   Some viruses other than HRV, such as Coxsakie A virus, also infect human host cells via ICAM-1. Therefore, the mvAbs against ICAM-1 can also offer protection against infection by these viruses. Such protective effect ofmvAbs can be demonstrated by cell protection assay as described above by using a minimum amount of viruses needed to yield a cytopathic effect within 24 to 48 hours.

### Example 2: Isolating human single chain antibodies (scFv against ICAM-1 and LDLR from a human scFv phage display library

Vaughan *et al*., (1996) Nature Biotechnology 14:309-314 have successfully isolated several human single chain antibodies with high affinities from a phage display library containing a repertoire of human scFv fragments. Human scFvs against ICAM-1 and LDLR can be isolated using similar strategy.
i) Construction of human scFv library is carried out as described in Vaughan *et al*., (1996) Nature Biotechnology 14:309-314.
ii) Isolating ICAM-1 and LDLR binding phage by panning
   Human ICAM-1 or LDLR is diluted to a concentration of 20µg/ml and is used to coat inimunotubes (Maxisorb, Nunc) by incubating for 1 h at 4°C. The remaining binding sites are saturated by bovine serum albumin (BSA). A portion of the amplified human scFv library (10¹³ phage) is first incubated for 2 h at 4°C in immunotubes precoated with 1 mg/ml BSA. The phages unbound to BSA are transferred to the immunotubes precbated with human ICAM-1 or LDLR After incubation for overnight at 4°C, the unbound phage are removed by washing 10 times with PBS buffer containing 0.5% Tween 20. The bound phages are eluted with 0.1M glycine buffer, pH 2.2, containing 1 mg/ml BSA. Four additional rounds are carried out in immunotubes coated with 5 µg/ml proteins. The panning protocols are also described in Griffiths *et al*., (1993) EMBO J. 12:725-734, and Hodits *et al*., (1995) J. Bio. Chem. 270:24078-24085, and Welply, J. K. *et al*., (1996) Proteins 26:262-270.
   Alternatively, mouse fibroblasts NIH3T3 which express human ICAM-1 or human LDLR will be established and used for panning. Specifically, a portion of the human scFv library is first incubated with parental NIH3T3 cells, the unbound phages are transferred to either NIH3T3-ICAM-1 cells or NIH3T3-LDLR cells. After incubation, the unbound phages are washed away, and the bound phages are amplified by infecting E. coli TG-1. The panning are performed for at least two more rounds.
iii) Isolating protective clones against HRV infection
   The ICAM-1 or LDLR binding phages are isolated from single ampicillin-resistant colonies of infected (suppressor) E. coli TG- I using helper phage VCSM 13 (Stratagene), and the phages are used to infect the (nonsuppressor) E. coli HB2151. Single ampicillin-resistant colonies are used to inoculated 200 µl of culture broth in microtiter plates, and the expression of soluble scFv fragments is induced by the addition of 1 mM isopropyl-b-D thiogalactopyranoside to the culture (Griffiths, A. D. *et al*.,(1993) EMBO J. 12:725-734, and Hodits *et al*., (1995) J. Bio. Chem. 270:24078-24085, and Welply *et al*., (1996) Proteins 26:262-270). Bacteria are pelleted, supernatants containing scFv are collected, sterilized by filtration, and added to HeLa cells in cell protection assays as described previously. Clones giving rise to scFv which can protect HeLa cells from the cytopathic effects caused by HRV infection are selected, the encoding scFv regions are amplified via polymerase chain reactions from single colonies (Nissim *et al.,* (1994) EMBO J. 13:692-698). The cloned human scFv genes are used to construct multivalent recombinant antibodies.
   Alternatively, phage particles that carry scFv against HRV binding sites on either ICAM-1 or LDLR can be administered to human directly in nasal sprays as a way to prevent and/or treat the common cold.

### Example 3: Identifying binding peptides to the target sequences in human ICAM-1 by random peptide library

Random peptide libraries are constructed in phage fUSE5 vector as described previously (Scott, J. K. and Smith, G.P. (1990) Science 249:386-390). Construction and amplification of the libraries are also described in details in Smith, G.P. (1992) Cloning in fUSE vectors. Feb 10, 1992 ed. Division of Biological Sciences, University of Missouri, CO. and in Smith, G.P. & Scott, J.K. (1993) Methods Enzymol. 217:228-257.

Peptides which binds to the target sequences in human ICAM-1 are selected by panning:
Each of the target peptides is synthesized by a synthesizer, is diluted in PBS to a concentration of 20mg/mi and is used to coat 3.5 cm wells by incubating for 1 h at 4°C. The remaining binding sites are saturated by bovine serum albumin (BSA). A portion of the amplified random peptide library is first incubated for 2 h at 4°C in a 3.5 cm well precoated with 1 mg/mi BSA in PBS and 1 mM MnC12. The phages unbound to BSA are transferred to a similar well precoated with a target peptide. After incubation for 1 h at 4°C, the-unbound phage are removed by washing 10 times with PBS buffer containing 0.5% Tween 20. The bound phage is eluted with 0.1M glycine buffer, pH 2.2, containing 1 mg/mi BSA and 0.1 mg/mi phenol red. The phages are amplified using the K91kan bacteria and partially purified by precipitation with polyethylene glycol (Smith, G.P. (1992) Cloning in fUSE vectors. Feb 10, 1992 ed. Division of Biological Sciences, University of Missouri, CO. and in Smith, G.P. & Scott, J.K. (1993) Methods Enzymol. 2 17:228-257). The panning is repeated for two more rounds.

Sequences carried by the selected phage are then determined using the Sequenase kit (United States Biochemical) with the primer 5'-CCCTCATAGTTAAGCGTAACG-3'(Koivunen, E. *et al*., (J. Bio. Chem. 268:20205-20210).

### Example 4: Identifying binding peptides to the target sequences in human ICAM-1 by molecular recognition theory and target complementary library technology (TCLT)

According to the molecular recognition theory, peptides with opposite hydropathic profiles bind to each other. One example is that a pair of peptides encoded by the sense and anti-sense strands of DNA bind to each other specifically (see Blalock, J.E. (1990) TIBTECH 8:140-144), and they are known as complementary peptides.

Complementary peptides to the target sequences in human ICAM-1 are encoded by the antisense strand of human ICAM-1 gene in either 5'-3' or 3'-5' orientation. Therefore, for each target peptide, there are two complementary sequences, and both have specific affinity for the target peptide. The sequences of the complementary peptides for each target sequence in human ICAM-1 are listed in the following:
Target No. 1: VCRHR & GHRCL
Target No.2: LGLVTG & RTLVGF
Target No.3: PVVPRQEQLL & FLNEDGPLLA
Target No.4: FSCSLPIR & GIPVSCRF

These complementary peptides (and their homologs) and peptides containing such may have the ability to bind to human ICAM-1 molecule at the target sequences. The affinity of a complementary peptide to its target sequence can be improved by optimizing the peptide sequence via a computer program, or by selecting peptides from a target complementary peptide library as described in Provisional Application Serial No. 60/083046.

### Example 5: Construction of multivalent peptides against human ICAM-1

One way of making a multivalent peptide is to link multiple copies of a single peptide or multiple copies of different peptides in tandem repeats to create molecules with the following structures:
[peptide A-(linker)-]ₙ (n >or= 2) or
[peptide A-(linker)-peptide B-(linker)-]ₙ (n >or= 2)

A linker can be of variable length and be composed by any amino acid residues. An alternative way of making a multivalent peptide is to link a single peptide or a peptide tandem repeat with a polymerization sequence derived from a coiled-coil protein. These molecules have the following structures:
peptide A-(hinge)--polymerization sequence--purification tag or [peptide A-(linker)-]ₙ―polymerization sequence--purification tag

A pentavalent peptide is preferred. It can be constructed in the same way as the pentavalent recombinant antibody as described above. Once the nucleotide sequence encoding a peptide is known, those skilled in the art will be able to construct a gene of multivalent peptide in a fashion as described above.

The characterization and production of the multivalent peptide are carried out in the same fashion as the multivalent recombinant antibodies.

### FORMULATION AND ADMINISTRATION

The antibodies of the present invention can be administered to a host alone, or in a pharmaceutical composition comprising the active compound and a carrier or excipient.

In accordance with this invention, the aforementioned multivalent recombinant antibodies can be administered to a subject for reducing or inhibiting rhinovirus infection. The antibodies are useful as a prophylaxis or means for treating disorders such as the common cold and AOM. The pharmaceutical compositions of the invention contain the antibodies in association with a compatible pharmaceutically acceptable carrier material.

Techniques for formulation arid administration may be found in Remington's Pharmaceutical Sciences 18th ed., Mack Publishing Co., Easton, PA (1990). Any conventional carrier material for topical administration can be utilized. Carriers or excipients can be used to facilitate administration of the compound, for example, to increase the solubility of the compound. Furthermore, the pharmaceutical preparations may contain other pharmaceutically active agents. Additional additives such as preservatives, stabilizers, emulsifying agents, buffers and the like may be added in accordance with accepted practices of pharmaceutical compounding.

Any conventional composition utilized for application to the nasal epithelium can be utilized in accordance with this invention. The aforementioned antibodies are preferably prepared as sprays, ointments, tinctures, creams, gels, solutions, lotions, suspensions, and the like. The pharmaceutical preparations may be sterilized and/or may contain adjuvants such as preservatives, stabilizers, wetting agents, emulsifiers, salts for varying the Osmotic pressure and/or buffers. They can be prepared by mixing the aforementioned active ingredient (*i*.*e*., a pharmaceutically effective amount of a multivalent recombinant antibody) with non-toxic, therapeutically inert, solid or liquid carriers customarily used in such preparations.

Preferably, the topical solutions are contained in a pressurized bottle attached to a hand pump and the mvAbs or multivalent peptide is delivered to the nasal and nasopharyngeal mucosa as aerosols.

## Claims

1. A multivalent recombinant antibody against ICAM-1 comprising three or more antigen binding domains for ICAM-1, wherein said antibody has an apparent affinity constant for ICAM-1 of no less than 10⁸ M⁻¹.

2. The multivalent recombinant antibody of claim 1 comprising four or more antigen binding domains for ICAM-1.

3. The multivalent recombinant antibody of claim 1 comprising five or more antigen binding domains for ICAM-1.

4. The multivalent recombinant antibody of claim 1 comprising three or more single chain Fv fragments against ICAM-1, wherein each of said single chain Fv fragment is linked to a polymerization domain.

5. A topical formulation for preventing rhinovirus infection, comprising:
a pharmaceutically effective amount of a multivalent recombinant antibody of any of claims 1 to 4, and
a pharmaceutically acceptable carrier.

6. Use of a multivalent recombinant antibody of any of claims 1 to 4, in the manufacture of a medicament for preventing the common cold in a host and which antibody is for administration to the nasal epithelium of said host.

7. Use of a multivalent recombinant antibody of any of claims 1 to 4 in the manufacture of a medicament for preventing acute otitis media in a host and which antibody is for administration to the nasal epithelium of said host.

## Patentansprüche

1. Multivalenter rekombinanter Antikörper gegen ICAM-1 umfassend drei oder mehr Antigen-bindende Domänen für ICAM-1, worin der Antikörper eine apparente Affinitätskonstante für ICAM-1 von nicht weniger als 10⁸ M⁻¹ aufweist.

2. Multivalenter rekombinanter Antikörper gemäß Anspruch 1, umfassend vier oder mehr Antigen-bindende Domänen für ICAM-1.

3. Multivalenter rekombinanter Antikörper gemäß Anspruch 1, umfassend fünf oder mehr Antigen-bindende Domänen für ICAM-1.

4. Multivalenter Antikörper gemäß Anspruch 1, umfassend drei oder mehr einzelkettige Fv-Fragmente gegen ICAM-1, worin jedes der einzelkettigen Fv-Fragmente mit einer Polymerisierungsdomäne verknüpft ist.

5. Topische Formulierung zum Vorbeugen einer RhinovirusInfektion, umfassend:
eine pharmazeutisch wirksame Menge eines mulitvalenten rekombinanten Antikörpers nach einem der Ansprüche 1 bis 4 und einem pharmazeutisch annehmbaren Träger.

6. Verwendung eines multivalenten rekombinanten Antikörpers nach einem der Ansprüche 1 bis 4 zur Herstellung eines Medikaments zur Vorbeugung einer gewöhnlichen Erkältung in einem Wirt, wobei der Antikörper zur Verabreichung über des nasale Epithel des Wirts vorgesehen ist.

7. Verwendung eines multivalenten rekombinanten Antikörpers nach einem der Ansprüche 1 bis 4 zur Herstellung eines Medikaments zur Vorbeugung einer akuten Mittelohrentzündung in einem Wirt, wobei der Antikörper zur Verabreichung über des nasale Epithel des Wirts vorgesehen ist.

## Revendications

1. Anticorps recombinant multivalent contre ICAM-1, comprenant trois ou plus de trois domaines de liaison d'antigène pour ICAM-1, ledit anticorps ayant une constante d'affinité apparente pour ICAM-1 non inférieure à 10⁸ M⁻¹.

2. Anticorps recombinant multivalent suivant la revendication 1, comprenant quatre ou plus de quatre domaines de liaison d'antigène pour ICAM-1.

3. Anticorps recombinant multivalent suivant la revendication 1, comprenant cinq ou plus de cinq domaines de liaison d'antigène pour ICAM-1.

4. Anticorps recombinant multivalent suivant la revendication 1, comprenant trois ou plus de trois fragments Fv monocaténaires contre ICAM-1, dans lequel chacun desdits fragments Fv monocaténaires est lié à un domaine de polymérisation.

5. Formulation topique pour la prévention d'une infection par un rhinovirus, comprenant :
une quantité pharmaceutiquement efficace d'un anticorps recombinant multivalent de l'une quelconque des revendications 1 à 4, et
un support pharmaceutiquement acceptable.

6. Utilisation d'un anticorps recombinant multivalent suivant l'une quelconque des revendications 1 à 4, dans la production d'un médicament destiné à la prévention du rhume chez un hôte, anticorps qui est destiné à l'administration à l'épithélium nasal dudit hôte.

7. Utilisation d'un anticorps recombinant multivalent suivant l'une quelconque des revendications 1 à 4, dans la production d'un médicament destiné à la prévention de l'otite moyenne aigue chez un hôte, anticorps qui est destiné à l'administration à l'épithélium nasal dudit hôte.
